# EUROPEAN PATENT SPECIFICATION

(11) **EP 3 338 762 B1**
(45) Date of publication and mention of the grant of the patent: **09.12.2026**
(21) Application number: 16306789.5
(22) Date of filing: 22.12.2016
(51) Int. Cl.: A61K 8/60, A61Q 19/00

(54) **USE OF RHAMNOLIPIDS FOR THE COSMETIC TREATMENT OF SKIN REDNESS**
VERWENDUNG VON RHAMNOLIPID ZUR BEHANDLUNG VON HAUTRÖTUNG
UTILISATION DE RHAMNOLIPID POUR TRAÎTER LA ROUGEUR DE LA PEAU

(43) Date of publication of application: 27.06.2018
(73) Proprietor: L'OREAL, 75008 Paris (FR)
(72) Inventor: GUENICHE, Audrey, BP22 93601 AULNAY-SOUS-BOIS (FR); TOURNIER-COUTURIER, Lucie, BP22 93601 AULNAY-SOUS-BOIS (FR)
(74) Representative: Lavoix

(56) References cited:
- EP-A1- 2 786 743
- EP-A2- 0 153 634
- WO-A2-2011/056871
- WO-A2-93/14767
- DE-A1- 4 127 908
- US-A- 5 455 232
- STIPCEVIC T ET AL: "Di-rhamnolipid from Pseudomonas aeruginosa displays differential effects on human keratinocyte and fibroblast cultures", JOURNAL OF DERMATOLOGICAL SCIENCE, ELSEVIER, AMSTERDAM, NL, vol. 40, no. 2, 1 November 2005 (2005-11-01), pages 141 - 143, XP027793006, ISSN: 0923-1811, [retrieved on 20051101]
- DATABASE GNPD [online] "MINTEL"; 10 July 2025 (2025-07-10), "Dream Bonds Bio Emulsion", XP093418373, retrieved from https://www.gnpd.com/sinatra/recordpage/13009998/ Database accession no. 13009998

## Description

The present invention concerns methods for the cosmetic treatment of skin redness.

Skin redness is a skin disorder, primarily present on the face, which can have various origins. It may be of short duration (flushes) or may be permanent.

This redness may be triggered by a wide variety of factors, such as the consumption of food or of hot or alcoholic beverages, by rapid temperature variations, by heat or cold, by a low relative humidity, by exposure of the skin to raging winds or to drafts (fans, air-conditioning), and by the application of surfactants or other compounds even when these are not known to be, especially, irritants.

These red blotches, which can persist for several hours after exposure of the skin to the stimulus, are often unaesthetic and often create significant psychological embarrassment.

It is now known that these cutaneous reactions are related in particular to a release of neuropeptides from the nerve endings of the epidermis and dermis. Additionally, skin redness is characterized by an abnormally high secretion of proinflammatory molecules which induce capillaries vasodilation.

Hence, important need still exists for an effective method of prevention or esthetic treatment of cutaneous redness.

The present inventors surprisingly demonstrated that particular rhamnolipids could reduce red blotches on the skin, in particular red blotches induced by external stimuli. Indeed, they showed that the application of particular rhamnolipids on a skin model stimulated by capsaicin, used as a model of skin redness, significantly decreased the percentage of capillaries dilated further to capsaicin stimulation and significantly decreased the surface of dilated vessels.

Rhamnolipids are biosurfactants secreted from *Pseudomonas aeruginosa* which provide great antibacterial and antifungal activity, which makes them an attractive alternative to chemical surfactants. They have been further proposed in the medical field to combat certain types of bacteria, viruses and fungi, or to repair skin injuries (see WO 2011/056871 A2). However, to the inventor's knowledge, no effect of these particular rhamnolipids on capillaries dilation induced by external stimuli, and thereby on skin redness, has been disclosed so far.

The invention is as defined by the claims.

The present disclosure thus concerns a method for the cosmetic treatment of skin redness induced by an external stimulus, comprising administering to a subject in need thereof an effective amount of at least one rhamnolipid.

The present invention relates to a non-therapeutic use of at least one rhamnolipid or a cosmetic composition comprising it, for the cosmetic treatment of skin redness induced by consumption of food or of hot or alcoholic beverages, by a low relative humidity, by exposure of the skin to raging winds or to drafts, by mechanical stress or by the application of surfactants

As used herein, the term "redness" or "red blotch" refers to a pink to red, or even dark red, coloration of all or part of the skin of the body, scalp, mucous membranes or semi-mucous membranes. This manifestation, also referred to as erythema, may be sign of a healthy complexion (pink cheeks), but, while benign, it is however sometimes undesirable and considered as unesthetical.

Redness is generally induced by external stimuli such as consumption of food or of hot or alcoholic beverages, by rapid temperature variations, by heat or cold, by a low relative humidity, by exposure of the skin to raging winds or to drafts (fans, air-conditioning), by mechanical stress (*e.g*. friction on contact with a razor) or by the application of surfactants or other compounds even when these are not known to be, especially, irritants, thereby leading to an irritated skin.

In a particular aspect of the disclosure, the external stimulus is chosen from heat, irritated skin and cold.

In the context of the invention, the term "skin" means any cutaneous surface of the body, preferentially the skin of the face.

As used herein, the term "treating" or "treatment" refers to any action that aims to improve the comfort or the well-being of an individual. This term therefore covers preventing, attenuating, relieving or suppressing skin redness, *i.e.* an unaesthetic disorder, but is limited to a cosmetic treatment.

In a particular embodiment, the at least one rhamnolipid is at least one rhamnolipid of formula (I) 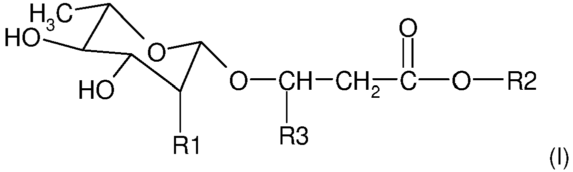 wherein
R₁ is selected from the group consisting of H and α-L-rhamnopyranosyl,
R₂ is selected from the group consisting of H, a C₁-C₆ linear or branched alkyl, -CH(R₄)-CH₂-COOH and CH(R₄)-CH₂-COOR₅,
R₃ is -(CH₂)ₓCH₃, where x is an integer from 4 to 12, preferably x is 6,
R₄ is -(CH₂)_{y}CH₃, where y is an integer from 1 to 10, preferably y is 4, 6 or 8, and
R₅ is a C₁-C₆ linear or branched alkyl, preferably -CH₃.

In a preferred embodiment, the at least one rhamnolipid is a mixture of rhamnolipids, in particular a mixture of rhamnolipids of formula (I) as defined above. The mixture of rhamnolipids may in particular comprise a mono-rhamnolipid, a di-rhamnolipid or a combination of both.

In a particular embodiment the at least one rhamnolipid is a mono-rhamnolipid of formula (I) 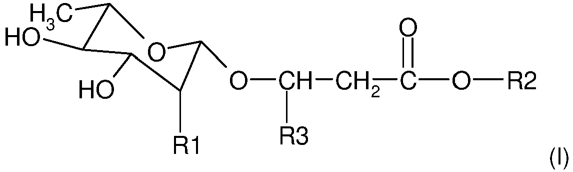 wherein
R₁ is H,
R₂ is selected from the group consisting of H, a C₁-C₆ linear or branched alkyl, -CH(R₄)-CH₂-COOH and CH(R₄)-CH₂-COOR₅,
R₃ is -(CH₂)ₓCH₃, where x is an integer from 4 to 12, preferably x is 6,
R₄ is -(CH₂)_{y}CH₃, where y is an integer from 1 to 10, preferably y is 4, 6 or 8, and
R₅ is a C₁-C₆ linear or branched alkyl, preferably -CH₃,
or their salts, isomers or solvates.

In a particularly preferred embodiment, the at least one rhamnolipid is a mono-rhamnolipid of formula (I) 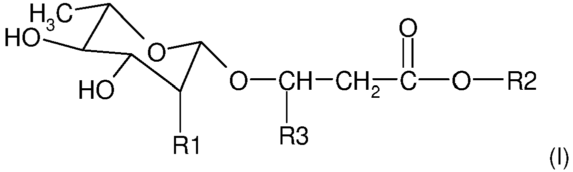 wherein
R₁ is H,
R₂ is -CH(R₄)-CH₂-COOH,
R₃ is -(CH₂)ₓCH₃, where x is an integer from 4 to 12, preferably x is 6, and
R₄ is -(CH₂)_{y}CH₃, where y is an integer from 1 to 10, preferably y is 4, 6 or 8,
or their salts, isomers or solvates.

In another particular embodiment the at least one rhamnolipid is a di-rhamnolipid of formula (I) 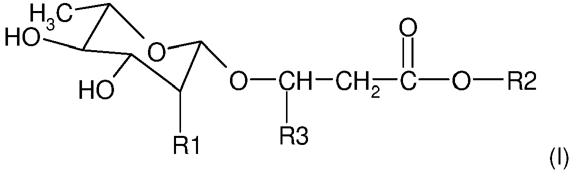 wherein
R₁ is α-L-rhamnopyranosyl,
R₂ is selected from the group consisting of H, a C₁-C₆ linear or branched alkyl, -CH(R₄)-CH₂-COOH and CH(R₄)-CH₂-COOR₅,
R₃ is -(CH₂)ₓCH₃, where x is an integer from 4 to 12, preferably x is 6,
R₄ is -(CH₂)_{y}CH₃, where y is an integer from 1 to 10, preferably y is 4, 6 or 8, and
R₅ is a C₁-C₆ linear or branched alkyl, preferably -CH₃,
or their salts, isomers or solvates.

In a particularly preferred embodiment, the at least one rhamnolipid is a di-rhamnolipid of formula (I) 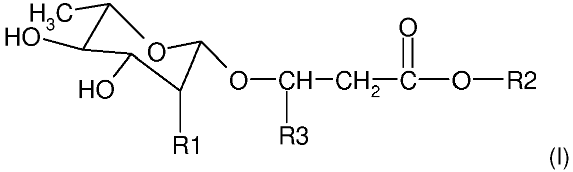 wherein
R₁ is α-L-rhamnopyranosyl,
R₂ is -CH(R₄)-CH₂-COOH,
R₃ is -(CH₂)ₓCH₃, where x is an integer from 4 to 12, preferably x is 6, and
R₄ is -(CH₂)_{y}CH₃, where y is an integer from 1 to 10, preferably y is 4, 6 or 8,
or their salts, isomers ou solvates.

In a particularly preferred embodiment, the at least one rhamnolipid is a mixture of at least one mono-rhamnolipid as defined above and at least one di-rhamnolipid as defined above.

In another embodiment, said at least one rhamnolipid is a mixture comprising at least one di-rhamnolipid of formula (II) 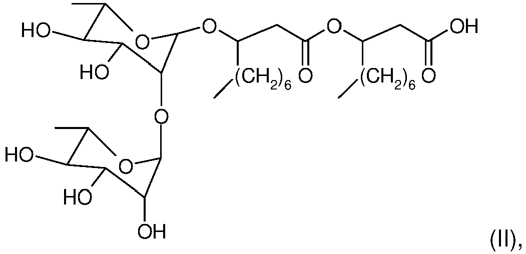 called DiC10C10,
or its salts, isomers or solvates,
and at least one mono-rhamnolipid of formula (III) 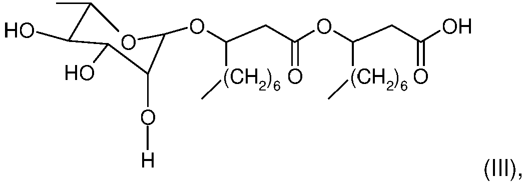 called MonoC10C10,
or its salts, isomers or solvates.

In another particular embodiment, said at least one rhamnolipid is a mixture comprising MonoC10C10 or its salts, isomers or solvates, DiC10C10 or its salts, isomers or solvates, the di-rhamnolipid of formula (IV) 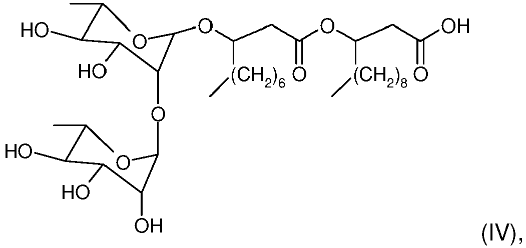 called DiC10C12,
or its salts, isomers or solvates,
and the di-rhamnolipid of formula (V) 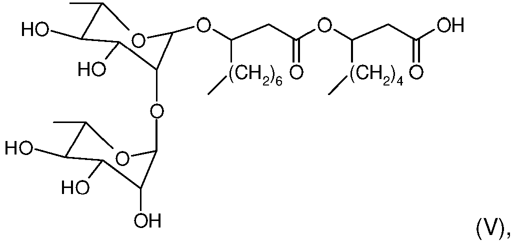 called DiC10C8,
or its salts, isomers or solvates.

In a particularly preferred embodiment, said at least one rhamnolipid is a mixture exclusively comprising, as rhamnolipids, MonoC10C10, DiC10C10, DiC10C12 and DiC10C8, or their salts, isomers or solvates.

In a particular embodiment, said mixture comprises:
- from 30% to 40% by weight of MonoC10C10, or its salts, isomers or solvates, in particular from 31% to 39%, from 32% to 38%, from 33% to 37%, from 34% to 36%, or from 35% to 35.5% by weight of MonoC10C10;
- from 45% to 55% by weight of DiC10C10, or its salts, isomers or solvates, in particular from 46% to 54%, from 47% to 53%, from 48% to 52%, from 49% to 51% or from 50% to 51% by weight of DiC10C10;
- from 5% to 15% by weight of DiC10C12, or its salts, isomers or solvates, in particular from 6% to 14%, from 7% to 13%, from 8% to 12%, from 9% to 11% or from 9.5% to 10% by weight of DiC10C12; and
- from 0.5% to 10% by weight of DiC10C8, or its salts, isomers or solvates, in particular from 1% to 9%, from 2% to 8%, from 3% to 7%, from 3.5% to 6% or from 4% to 5% by weight of DiC10C8;
wherein the percentage by weight refers to the weight of said rhamnolipid calculated on the acidic form (COOH) compared to the total weight of rhamnolipids in the mixture calculated on the acidic form (COOH).

In a particularly preferred embodiment, said mixture comprises:
- from 34% to 36% by weight of MonoC10C10;
- from 48% to 52% by weight of DiC10C10;
- from 9% to 11% by weight of DiC10C12; and
- from 3% to 7% by weight of DiC10C8;
wherein the percentage by weight refers to the weight of said rhamnolipid calculated on the acidic form (COOH) compared to the total weight of rhamnolipids in the mixture calculated on the acidic form (COOH).

The carboxylic function of rhamnolipids described above can be present as carbocylate salt with an organic or inorganic cation. The cation is preferably chosen among sodium, potassium, calcium and ammonium.

As the cation provides electronuetrality to the molecule, it is understood that, when the cation comprises several cationic charges, then the same cation can provide electroneutrality for several anionic groups such as carbocylates in a same molecule or can provide electroneutrality for several molecules.

The rhamnolipids used in the context of the invention may be used as mixt or highly purified rhamnolipids. A mixt rhamnolipid is a rhamnolipid, having other components as Glycerid residues and/or a variety of various rhamnolipid mixtures. Highly purified rhamnolipid is a rhamnolipid whose external impurities have been removed and/or wherein the rhamnolipid have been purified from the other various rhamnolipids present in the crude form.

As well-known from the skilled person, rhamnolipids are secreted by bacteria. Bacteria capable of synthesizing the rhamnolipids used in the context of the present invention can be isolated from oil environnements, which have been found to contain bacteria which produce rhamnolipids when the bacteria are grown on either a soluble carbon nutrient source (glucose) or an insoluble carbon nutrient source (glycerol, gas oil). In particular, the rhamnolipids used in the context of the invention may be produced by *Pseudomonas aeruginosa.*

Accordingly, in a particular embodiment, said at least one rhamnolipid is included in or in the form of a bacterial extract, *i.e.* is in crude form. Preferably, said at least one rhamnolipid is included in or in the form of a *Pseudomonas aeruginosa* bacterial extract.

In the context of the invention, the term "bacterial extract" refers both to a set of compounds produced and secreted by a bacterium, thus present in the culture medium of the bacterial culture (and therefore in the culture supernatant of the bacterial culture after centrifugation), and a set of compounds comprised in the bacterium, thus present in the bacterial pellet of the bacterial culture after centrifugation. Preferably, the bacterial extract refers to the culture medium of the bacterial culture, including compounds secreted by the bacterium.

The inventors demonstrated that the rhamnolipids produced by the *Pseudomonas aeruginosa* strain PA1, disclosed in Santa Anna et al. (2002) Braz. J. Chem. Eng. 19:159-166, were particularly efficient in the treatment of skin redness.

Accordingly, in a particular embodiment, said at least one rhamnolipid is included in or in the form of a bacterial extract from *Pseudomonas aeruginosa* strain PA1.

The rhamnolipids used in the context of the invention may be prepared by any conventional method well-known from the skilled person. The bacterial extract containing the rhamnolipids used in the context of the invention may in particular be obtained from a culture of at least one strain of *Pseudomonas aeruginosa,* preferably from the *Pseudomonas aeruginosa* strain PA1.

Conventional methods to prepare rhamnolipids from producing bacteria, in particular to prepare bacterial extracts comprising rhamnolipids, are well-known from the skilled person and typically comprise fermentation, isolation and purification as described in U.S patent 5,455,232 and US 5,466,675, in Santa Anna et al. (2002) Braz. J. Chem. Eng. 19:159-166 or in Dos Santos et al. (2016) PeerJ 4:e2078.

Typically, the bacterium *Pseudomonas aeruginosa* is cultured in a suitable medium and grown to a desired density. Preferably, the bacterial themselves are removed from the culture media by any method known in the art, such as centrifugation. The supernatant may then be used directly as the crude preparation, or further processing steps well-known from the skilled person may be carried out such as concentration, dehydration, filtration, purification, column chromatography, and the like. Preferably, the final rhamnolipid preparation is not highly purified and corresponds to a crude preparation, preferably comprising a mixture of mono-rhamnolipids and di-rhamnolipids as defined above.

In a particular embodiment, the bacterial extract containing the rhamnolipids used in the context of the invention is obtained by fermenting at least one strain of *Pseudomonas aeruginosa,* preferably the strain *Pseudomonas aeruginosa* PA1, in a culture medium, separating the supernatant comprising the at least one rhamnolipid from the bacterial cells, and optionally at least partially dehydrating, sterilizing, purifying, grinding and/or acylating the bacterial extract thus obtained.

Preferably, the process of preparation of the rhamnolipids used in the context of the invention is carried out so that the rhamnolipids represent 40% to 65% by weight of the total dry matter weight of the bacterial extract.

Accordingly, in a particular embodiment, the at least one rhamnolipid represents 40% to 65% by weight of the total dry matter weight of the bacterial extract, preferably 45% to 60% by weight of the total dry matter weight of the bacterial extract.

As used herein, the term "effective amount" refers to the amount of the rhamnolipid, that, as a whole, enables treating skin redness.

According to the present invention, the process is a non-therapeutic process.

Preferably, the at least one rhamnolipid used in the context of the invention is contained in a cosmetic composition further comprising a cosmetically acceptable carrier.

By "cosmetically acceptable carrier" is meant herein a non-toxic carrier which can be applied to the skin.

Preferably, the at least one rhamnolipid is comprised in the cosmetic composition at a concentration of 0.0001% to 30% by weight compared to the total weight of the composition, in particular at a concentration of 0.01% to 15% by weight, more particularly of 0.1% to 10% by weight compared to the total weight of the composition.

The present disclosure also relates to a composition, in particular a cosmetic composition, comprising at least one rhamnolipid as defined above.

In a particular embodiment, said composition comprises a mixture comprising or exclusively comprising, as rhamnolipids, MonoC10C10, DiC10C10, DiC10C12 and DiC10C8, as defined above, or their salts, isomers or solvates.

In a particular aspect disclosed herein, said mixture comprises:
- from 30% to 40% by weight of MonoC10C10, or its salts, isomers or solvates, in particular from 31% to 39%, from 32% to 38%, from 33% to 37%, from 34% to 36%, or from 35% to 35.5% by weight of MonoC10C10;
- from 45% to 55% by weight of DiC10C10, or its salts, isomers or solvates, in particular from 46% to 54%, from 47% to 53%, from 48% to 52%, from 49% to 51% or from 50% to 51% by weight of DiC10C10;
- from 5% to 15% by weight of DiC10C12, or its salts, isomers or solvates, in particular from 6% to 14%, from 7% to 13%, from 8% to 12%, from 9% to 11% or from 9.5% to 10% by weight of DiC10C12; and
- from 0.5% to 10% by weight of DiC10C8, or its salts, isomers or solvates, in particular from 1% to 9%, from 2% to 8%, from 3% to 7%, from 3.5% to 6% or from 4% to 5% by weight of DiC10C8;
wherein the percentage by weight refers to the weight of said rhamnolipid calculated on the acidic form (COOH) compared to the total weight of rhamnolipids in the mixture calculated on the acidic form (COOH).

In a particularly aspect disclosed herein, said mixture comprises:
- from 34% to 36% by weight of MonoC10C10;
- from 48% to 52% by weight of DiC10C10;
- from 9% to 11% by weight of DiC10C12; and
- from 3% to 7% by weight of DiC10C8;
wherein the percentage by weight refers to the weight of said rhamnolipid calculated on the acidic form (COOH) compared to the total weight of rhamnolipids in the mixture calculated on the acidic form (COOH).

The composition according to the invention comprises a mixture of rhamnolipids,
wherein the mixture comprises:
- from 31% to 39% by weight of MonoC10C10 or its salts, isomers or solvates,
- from 45% to 55% by weight of DiC10C10 or its salts, isomers or solvates,
- from 5% to 15% by weight of DiC10C12 or its salts, isomers or solvates, and
- from 0.5% to 10% by weight of DiC10C8 or its salts, isomers or solvates,
wherein the percentage by weight refers to the weight of said rhamnolipid calculated on the acidic form (COOH) compared to the total weight of rhamnolipids in the mixture calculated on the acidic form (COOH).

In another particular embodiment, said composition comprises a mixture of rhamnolipids as defined above, included in or in the form of a *Pseudomonas aeruginosa* bacterial extract, as defined above.

The composition of the invention may be in any galenic form normally used in the cosmetic field. In particular, the composition may be in the form of a powder, *i.e.* in dry form, or in the form of a suspension, an emulsion or a solution.

The cosmetically acceptable carrier may be of diverse nature depending on the type of composition considered.

In particular, in the case of compositions intended for topical application, the composition may be in the form of an aqueous solution, an aqueous-alcoholic or oily solution, a lotion- or serum-type dispersion, emulsions of liquid or semi-liquid consistency of the milk type, a cream-type suspension or emulsion, an anhydrous or aqueous gel, microemulsions, microcapsules, microparticles, or vesicular dispersions of ionic and/or nonionic type.

These compositions can be formulated according to the usual techniques.

These compositions in particular constitute a cleansing, protective or care cream for the face, for the hands, for the feet, for the major anatomical folds or for the body (for example day creams, night creams, makeup-removing creams, foundation creams), makeup products such as fluid foundations, makeup-removing milks, protective or care body milks, aftersun milks, skincare lotions, gels or mousses, as cleansing lotions, bath compositions, deodorizing compositions containing a bactericidal agent, aftershave lotions or gels or hair-removing creams.

These compositions can also be formulated as a solid preparation constituting a cleansing bar or a soap.

When the composition is an emulsion, the proportion of the fatty phase may range from 5% to 80% by weight and preferably from 8% to 50% by weight relative to the total weight of the composition. The oils, emulsifiers and coemulsifiers may be present in a proportion ranging from 0.3% to 30% by weight and preferably from 0.5% to 20% by weight relative to the total weight of the composition.

When the composition is an oily solution or gel, the fatty phase may represent more than 90% of the total weight of the composition.

As well-known from the skilled person, the formulations for topical application can also contain adjuvants that are common in the cosmetic field, such as hydrophilic or lipophilic gelling agents, hydrophilic or lipophilic active agents, preservatives, antioxidants, solvents, fragrances, bactericides, odor adsorbing agents, dyestuffs and colorants. The amounts of these various adjuvants are those conventionally used in the particular field, and, for example, range from 0.01% to 20% of the total weight of the composition. Depending on their nature, these adjuvants can be introduced into the fatty phase and/or into the aqueous phase.

As an example, fatty materials that can be used according to the invention, include mineral oils such as hydrogenated polyisobutene and vaseline oil, plant oils such as a liquid fraction of karite butter, sunflower oil and apricot seed oil, animal oils such as perhydrosqualene, synthetic oils such as purcellin oil, isopropyl myristate and hexyl ethyl palmitate, unsaturated fatty acids, and fluoro oils such as perfluoropolyethers. Fatty alcohols and fatty acids such as stearic acid can also be used. Waxes such as paraffin, carnauba and beeswax can further be used. It is also possible to use silicone oils such as cyclomethicone and dimethicone, and siliconed waxes, resins and gums.

As an example, emulsifiers usable in the compositions of the invention include glyceryl stearate, polysorbate 60, the mixture cetylstearyl alcohol/cetylstearyl alcohol oxyethylenated with 33 moles of ethylene oxide marketed under the trademark Sinnowax AO^{®} by Henkel, the mixture PEG-6/PEG-32/glycol stearate marketed under the trademark Tefose^{®}63 by Gattefosse, PPG-3 myristyl ether, siliconed emulsifiers such as cetyldimethicone copolyol and sorbitan mono- or tristearate, PEG-40 stearate and oxyethylenated sorbitan monostearate (20OE).

Representative solvents which can be used include the lower alcohols, in particular ethanol and isopropanol, propylene glycol.

As an example, hydrophilic gelling agents include carboxylic polymers such as carbomer, acrylic copolymers such as acrylate/alkylacrylate copolymers, polyacrylamides in particular the mixture of polyacrylamide, C13-14-isoparaffin and Laureth-7 marketed under the trademark Sepigel 305^{®} by Seppic, polysaccharides such a cellulose derivatives including hydroxyalkylcelluloses, in particular hydroxypropylcellulose and hydroxyethylcellulose, natural gums such as guar, carob and xanthan, and clays.

As an example, lipophilic gelling agents include modified clays such as bentones, metal salts of fatty acids such as aluminum stearates, and hydrophobic silica, or still ethylcellulose and polyethylene.
Of course, the compositions of the invention can additionally contain several other compounds.

In a preferred embodiment, the compositions used in the context of the invention are for topical administration.

Preferably, in the methods disclosed herein, the at least one rhamnolipid is administered topically. Preferably, the at least one rhamnolipid is applied on the face, the hands, the feet, the major anatomical folds or the body, more preferably on the face.

The method disclosed herein may comprise a single administration. In another embodiment, the administration is repeated for example 2 to 3 times daily for a day or more and generally for an extended period of at least 4 weeks or 4 to 15 weeks, or as much as necessary with possible break if needed.

In the whole description, the expression « comprising a » or « containing a » means « comprising at least one » or « containing at least one », unless otherwise specified.

In the description and in the following examples, unless stated otherwise, the percentages are percentages by weight and the ranges of values written as "between ... and ..." include the upper and lower limits specified. The ingredients are mixed, before their shaping, in order and under conditions easily determined by the skilled person.

The present invention will be further illustrated by the examples below.

### Examples

### Example 1: Production of rhamnolipids from Pseudomonas aeruginosa strain PA1.

This example describes the production of the rhamnolipids used in Example 2.

### 1. Microorganism maintenance and preinoculum preparation

The PA1 strain of *Pseudomonas aeruginosa,* disclosed in Santa Anna et al. (2002) Braz. J. Chem. Eng. 19:159-166**,** was preserved in 10 % glycerol in an ultrafreezer at -80°C. The preinoculum was grown on a plate with YPDA (yeast extract 0.3%, peptone 1.5%, dextrose 0.1%, agar 1.2%) at 30°C for 48 hours and was transferred to 1000 ml flasks with 300 ml of medium of the following composition (g/I): NaNO₃ 1.0, KH₂PO₄ 3.0, K₂HPO₄ 7.0, MgSO₄.7H₂O 0.2, yeast extract 5.0, peptone 5.0 and glycerol P.A. 30.0. After 24 hours of cultivation, the fermentation medium containing the cells was stored in cryotubes with a glycerol/fermentation medium ratio of 1:3 to act as standard preinoculum in all fermentations.

### 2. Preinoculum preparation

1.0 ml of the contents of a cryotube was inoculated into 300 ml of fermentation medium of the following composition (g/l): glycerol 30.0, NaNO₃ 1.0, K₂HPO₄ 7.0, KH₂PO₄ 3.0, MgSO₄.7H₂O 0.2, yeast extract 5.0 and peptone 5.0. The flasks were then incubated on rotary agitators at 30°C and 170 rpm for 40 hours. At the end of this period the cells from each flask were recovered by centrifuging (5000 g for 20 minutes) and utilised as inoculum in the bioreactors.

### 3. Sterilisation

The fermenter, containing the culture medium to be utilised, was autoclave-sterilized at 121°C for 15 minutes prior to each production run. The oxygenation system was sterilised by circulating a 1.0 % solution of sodium hypochlorite for 1 hour. Following this procedure, sterile distilled water was circulated through the system to eliminate traces of chlorine. Only then was inoculation of the microorganisms carried out.

### 4. Production of rhamnolipids by Pseudomonas aeruginosa PA1

The culture medium utilised in the fermentations had the following composition (g/l): glycerol 30.0, NaNO₃ 1. in 4, K₂HPO₄ 7.0, KH₂PO₄ 3.0 and MgSO₄.7H₂O 0.2, there resulting a carbon/nitrogen ratio of 60.

The principal fermentations were carried out in a BioFlo Ilc bioreactor (Batch/Continuous Fermenter, New Brunswick Scientific, USA) of 5.0 litres nominal capacity located in a fume cabinet with extraction. The average working volume utilised in the fermentations was 3.0 litres. The temperature was maintained at 30°C with agitation at 100 rpm. Oxygenation was carried out non-dispersively by means of a gas/liquid contactor.

Oxygenation was carried out with compressed air or employing a cylinder of pure oxygen. Oxygenation conditions were initially defined in accordance with the results of the oxygenation tests with the module utilised.

### 5. Sterilization and cell removal

After the fermentation (7-9 days, in simple batches), the suspension was sterilized in an autoclave at 121°C for 15 min. The bacterial cells were removed by centrifugation, at 10,000 rpm for 20 minutes.

### 6. Lyophilization

The solution containing the biosurfactants was then concentrated. Water was removed by lyophilization.

### 7. Rhamnolipid quantification and analytical data of the samples

The rhamnolipid quantification was carried out in an indirect way, using rhamnose as a reference - rhamnose is a product of the acid hydrolysis of the rhamnolipids. A method adapted from the one described by Pham et al. (2004) Microbiology 150:3405-3413, was used - the extraction step was suppressed.

The biosurfactant solution produced by this strain of *Pseudomonas aeruginosa* was also characterized by HPLC. A solution of H₂SO₄ was added to the biosurfactant solution until pH 2.0 was achieved, necessary for the precipitation of the rhamnolipids. The precipitated material was then dissolved in a chloroform:ethanol (2:1) solution and filtered. After the evaporation of the solvent, the purified rhamnolipids were derivatized and analyzed in the HPLC, using the patterns reported by Mata-Sandoval et al. (1999) Journal of Chromatography 864:211-220 as a reference.

The average composition of a rhamnolipid type biosurfactant solution produced as shown above is shown in Table 1.

**Table 1: Composition of a rhamnolipid solution.**

| **Rhamnolipid type** | **Molar mass (g/mol)** | **Mass concentration (%)** |
|---|---|---|
| MonoC10C10 | 492 | 35.3 |
| DiC10C10 | 638 | 51.0 |
| DiC10C12 | 666 | 9.8 |
| DiC10C8 | 610 | 3.9 |

The lyophilized extract obtained as shown above displayed the following specification:
Rhamnolipid content: 50-65%
Glycerol residue: 4-105%
Protein residue: 3-10%
Ashes: 15%

### Example 2: Effect of rhamnolipids on capillaries dilation

This example demonstrates the effect of rhamnolipids on capillaries dilation induced in a skin model of red blotches.

Capillaries dilation is a manifestation which is observed in skin redness after contact with a particular stimulus which will leads to this discomfort.

Capsaicin can be used as discomfort inducer. Indeed, in skins prone to redness, it is observed, after capsaicin application, burning sensations, pulling sensations, stinging sensations and/or red blotches, to different stimuli such as application of cosmetic or dermatologic products or soap.

This example demonstrates the modulation of capillaries vasodilation induced by rhamnolipids in a human skin model maintained in survival after capsaicin stimulation.

### Material and methods

### 1. Skin preparation

8 human skins from different donors were obtained from women (aged 30 to 55 years) after plastic surgery of the scalp and maintained in survival *ex vivo.*

Skin fragments were put in inserts which were in suspension above a culture well. Culture medium (DMEM including antibiotics and FCS) was added in the well, a passage occurring by slow diffusion between the two compartments through a porous membrane (3 µm). 5 hour-equilibration was carried out before the beginning of the experiment.

### 2. Experimental model of capillaries dilation using capsaicin and application of the 4 products

After the 5-hour equilibration, the experimental capillaries dilation model was performed by adding 10 µM of capsaicin in the culture medium. Simultaneously, a product comprising the rhamnolipid mixture obtained in Example 1 was tested at 2 concentrations. Skin fragments were maintained in organ culture for 24 hours in a humid atmosphere chamber at 37°C and 5% CO₂.

A comparative study was then carried out:
- Control skin (basal condition: non-stimulated, non-treated skin)
- Skin stimulated with capsaicin at 10 µM (inflammatory condition)
- Skin stimulated with capsaicin and treated with the rhamnolipid mixture of Example 1 at 0.5% and 0.1%.

### 3. Analysis

### - Histologic evaluation of capillaries diameter modifications

Skin fragments were fixed in formalin and included in paraffin. After coloration with hemalun-eosin, the capillaries caliber was studied.

After coloration with hemalun-eosin, the vascular dilation was studied by counting the number of dilated vessels on the whole histological section (16 fields at magnification x40). Thus number was reported to the total number of vessels in order to calculate the percentage of dilated vessels.

Furthermore, a morphometric analysis of the surface occupied by the vessels lumen was carried out in order to determine the mean surface (µm²) occupied by the vessels.

### - Statistical analysis

A mean was calculated from the results obtained on the 8 skins. The statistical analysis was performed with the Student test with a risk α of 5%.

### Results

### 1. Evaluation of the global percentage of dilated capillaries

The results concerning the global percentage of dilated capillaries are shown in Table 2 (mean±standard error).

**Table 2: Histologic evaluation of the percentage of dilated dermal capillaries**

| | Percentage |
|---|---|
| Control skin | **69.67** ± 11.4 |
| Skin + Capsaicin | **90.2** ± 3.7 |
| | * p = 0.0005 |
| Skin + Capsaicin + Rhamnolipids at 0.5% | **68.7** ± 16.8 |
| | # p = 0.01 |
| Skin + Capsaicin + Rhamnolipids at 0.1% | **66.4** ± 17.5 |
| | # p = 0.019 |

| | |
|---|---|
| * : statistically significantly different from the control skin (Student's test, p < 0.05) # : statistically significantly different from the Capsaicin condition (Student's test, p < 0.05) | |

Applying capsaicin (n = 8) induced a significant vasodilation compared to the control skin: *90.2% vs.* 69.7% (p = 0.0005).

Furthermore, a significant decrease of the percentage of dilated vessels was observed with the rhamnolipids, with 68.7% for the concentration 0.5% (p = 0.01, n = 6) and 66.4% for the concentration 0.1% (p = 0.02, n = 6).

### 2. Measurement of the mean capillaries surface

The results concerning the measurement of the mean capillaries surface are shown in Table 3 (mean±standard error).

**Table 3: Measurement of the surface (µm²) of the dilated capillaries**

| | Mean surface (µm²) |
|---|---|
| Control skin | **87.2** ± 24.4 |
| Skin + Capsaicin | **203.2** ± 64.5 |
| | * p = 0.001 |
| Skin + Capsaicin + Rhamnolipids at 0.5% | **90.3** ± 39.6 |
| | # p = 0.028 |
| Skin + Capsaicin + Rhamnolipids at 0.1% | **110.4** ± 60.4 |
| | # p = 0.04 |

| | |
|---|---|
| * : statistically significantly different from the control skin (Student's test, p < 0.05) # : statistically significantly different from the Capsaicin condition (Student's test, p < 0.05) | |

Applying capsaicin (n = 8) induced a significant vasodilation compared to the control skin: 203.2 µm² *vs.* 87.2 µm² (p = 0.001).

Furthermore, a significant decrease of the dilated vessels surface was observed with the rhamnolipids, with 90.3 µm² for the concentration 0.5% (p = 0.03, n = 6) and 110.4 µm² for the concentration 0.1% (p = 0.04, n = 6).

These results thus demonstrate that, in a model of human skin maintained in survival, an effect of rhamnolipids, used at 0.5% and 0.1%, was observed on vasodilation, a symptom of skin redness.

## Claims

1. Non-therapeutic use of at least one rhamnolipid or a cosmetic composition comprising it, for the cosmetic treatment of skin redness induced by consumption of food or of hot or alcoholic beverages, by a low relative humidity, by exposure of the skin to raging winds or to drafts, by mechanical stress or by the application of surfactants.

2. The non-therapeutic use according to claim 1, wherein said at least one rhamnolipid is at least one rhamnolipid of formula (I) 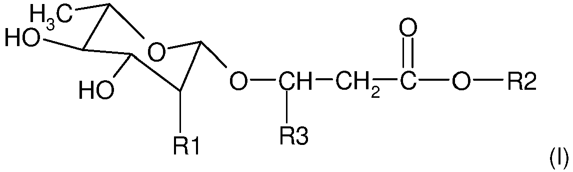 wherein
R₁ is selected from the group consisting of H and α-L-rhamnopyranosyl,
R₂ is selected from the group consisting of H, a C₁-C₆ linear or branched alkyl, -CH(R₄)-CH₂-COOH and CH(R₄)-CH₂-COOR₅,
R₃ is -(CH₂)ₓCH₃, where x is an integer from 4 to 12, preferably x is 6,
R₄ is -(CH₂)_{y}CH₃, where y is an integer from 1 to 10, preferably y is 4, 6 or 8, and
R₅ is a C₁-C₆ linear or branched alkyl, preferably -CH₃.

3. The non-therapeutic use according to claim 1 or 2, wherein said at least one rhamnolipid is a mixture of rhamnolipids, said mixture comprising at least one di-rhamnolipid of formula (II) 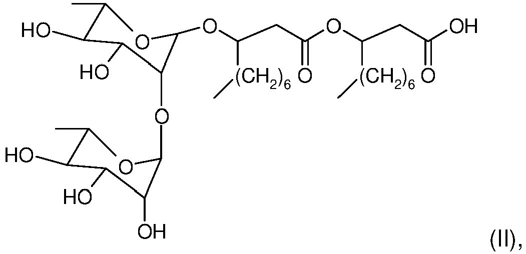 called DiC10C10,
or its salts, isomers or solvates,
and at least one mono-rhamnolipid of formula (III) 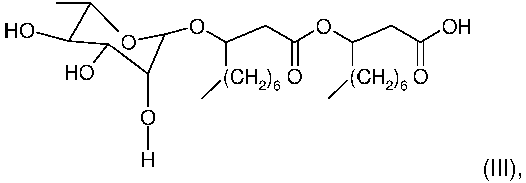 called MonoC10C10,
or its salts, isomers or solvates.

4. The non-therapeutic useaccording to claim 3, wherein said at least one rhamnolipid is a mixture comprising MonoC10C10 or its salts, isomers or solvates, DiC10C10 or its salts, isomers or solvates, the di-rhamnolipid of formula (IV) 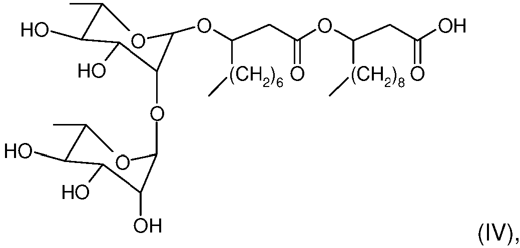 called DiC10C12,
or its salts, isomers or solvates,
and the di-rhamnolipid of formula (V) 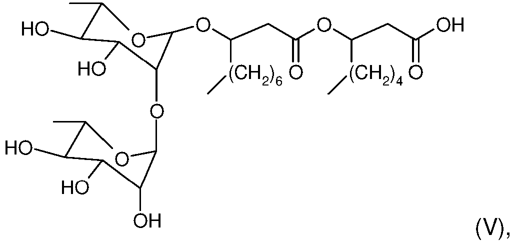 called DiC10C8,
or its salts, isomers or solvates.

5. The non-therapeutic use according to any one of claims 3 to 4, wherein the mixture comprises:
- from 30% to 40% by weight of MonoC10C10 or its salts, isomers or solvates,
- from 45% to 55% by weight of DiC10C10 or its salts, isomers or solvates,
- from 5% to 15% by weight of DiC10C12 or its salts, isomers or solvates, and
- from 0.5% to 10% by weight of DiC10C8 or its salts, isomers or solvates,
wherein the percentage by weight refers to the weight of said rhamnolipid calculated on the acidic form (COOH) compared to the total weight of rhamnolipids in the mixture calculated on the acidic form (COOH).

6. The non-therapeutic use according to any one of claims 1 to 5, wherein the at least one rhamnolipid is included in a bacterial extract.

7. The non-therapeutic use according to claim 6, wherein the at least one rhamnolipid represents 40% to 65% by weight of the total dry matter weight of the bacterial extract.

8. The non-therapeutic use according to claim 6 or 7, wherein the bacterial extract is obtained from a culture of at least one strain of *Pseudomonas aeruginosa.*

9. The non-therapeutic use according to any one of claims 6 to 8, wherein the bacterial extract is obtained by fermenting at least one strain of *Pseudomonas aeruginosa* in a culture medium, separating the bacterial extract comprising the at least one rhamnolipid from the bacterial cells, and optionally at least partially dehydrating, sterilizing, purifying, grinding and/or acylating the bacterial extract.

10. The non-therapeutic use according to any one of claims 1 to 9, wherein the at least one rhamnolipid is contained in a cosmetic composition further comprising a cosmetically acceptable carrier.

11. The non-therapeutic use according to claim 10, wherein the at least one rhamnolipid is comprised in the cosmetic composition at a concentration of 0.0001% to 30% by weight compared to the total weight of the composition.

12. The non-therapeutic use according to any one of claims 1 to 11, wherein said use is topical.

13. Composition comprising a mixture of rhamnolipids,
wherein the mixture comprises:
- from 31% to 39% by weight of MonoC10C10 or its salts, isomers or solvates,
- from 45% to 55% by weight of DiC10C10 or its salts, isomers or solvates,
- from 5% to 15% by weight of DiC10C12 or its salts, isomers or solvates, and
- from 0.5% to 10% by weight of DiC10C8 or its salts, isomers or solvates,
wherein the percentage by weight refers to the weight of said rhamnolipid calculated on the acidic form (COOH) compared to the total weight of rhamnolipids in the mixture calculated on the acidic form (COOH).

## Patentansprüche

1. Nichttherapeutische Verwendung von mindestens einem Rhamnolipid oder einer kosmetischen Zusammensetzung, die es umfasst, zur kosmetischen Behandlung von Hautrötungen, die durch den Verzehr von Lebensmitteln oder von heißen oder alkoholischen Getränken, durch eine niedrige relative Luftfeuchtigkeit, durch Aussetzen der Haut gegenüber heftigen Winden oder Zugluft, durch mechanische Belastung oder durch die Anwendung von Tensiden hervorgerufen werden.

2. Die nichttherapeutische Verwendung nach Anspruch 1, wobei das genannte mindestens eine Rhamnolipid mindestens ein Rhamnolipid der Formel (I) ist 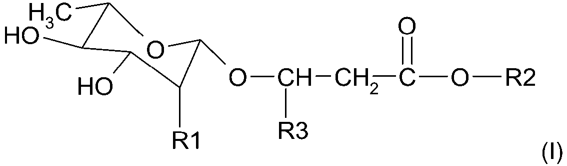 wobei
R₁ ausgewählt ist aus der Gruppe bestehend aus H und α-L-Rhamnopyranosyl,
R₂ ausgewählt ist aus der Gruppe bestehend aus H, einem linearen oder verzweigten C₁-C₆-Alkyl, -CH(R₄)-CH₂-COOH und CH(R₄)-CH₂-COOR₅,
R₃ -(CH₂)ₓCH₃ ist, wobei x eine ganze Zahl von 4 bis 12 ist, vorzugsweise x 6 ist,
R₄ -(CH₂)_{y}CH₃ ist, wobei y eine ganze Zahl von 1 bis 10 ist, vorzugsweise y 4, 6 oder 8 ist, und
R₅ ein lineares oder verzweigtes C₁-C₆-Alkyl ist, vorzugsweise -CH₃.

3. Die nichttherapeutische Verwendung nach Anspruch 1 oder 2, wobei das genannte mindestens eine Rhamnolipid eine Mischung von Rhamnolipiden ist, wobei die genannte Mischung mindestens ein Di-Rhamnolipid der Formel (II) umfasst 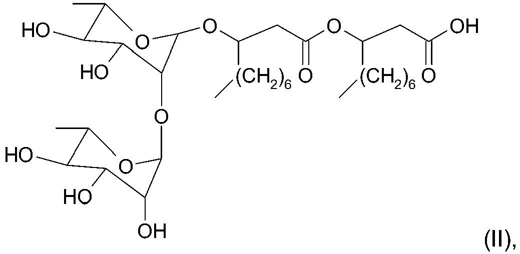 genannt DiC10C10,
oder dessen Salze, Isomere oder Solvate, und mindestens ein Mono-Rhamnolipid der Formel (III) 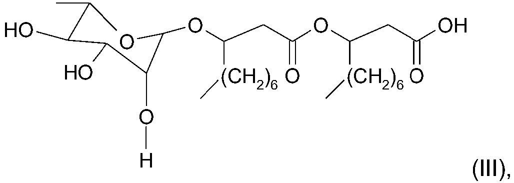 genannt MonoC10C10,
oder dessen Salze, Isomere oder Solvate.

4. Die nichttherapeutische Verwendungnach Anspruch 3, wobei das genannte mindestens eine Rhamnolipid eine Mischung ist, umfassend MonoC10C10 oder dessen Salze, Isomere oder Solvate, DiC10C10 oder dessen Salze, Isomere oder Solvate, das Di-Rhamnolipid der Formel (IV) 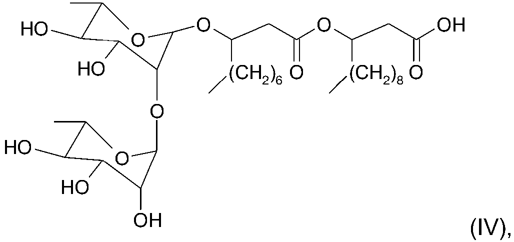 genannt DiC10C12,
oder dessen Salze, Isomere oder Solvate,
und das Di-Rhamnolipid der Formel (V) 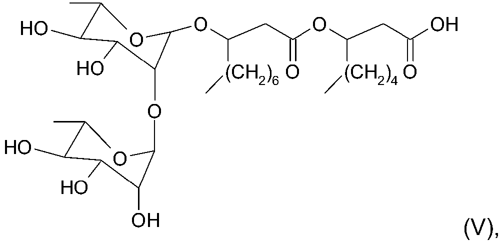 genannt DiC10C8, oder dessen Salze, Isomere oder Solvate.

5. Die nichttherapeutische Verwendung nach einem beliebigen der Ansprüche 3 bis 4, wobei die Mischung umfasst:
- von 30 % bis 40 Gewichtsprozent MonoC10C10 oder dessen Salze, Isomere oder Solvate,
- von 45 % bis 55 Gewichtsprozent DiC10C10 oder dessen Salze, Isomere oder Solvate,
- von 5 % bis 15 Gewichtsprozent DiC10C12 oder dessen Salze, Isomere oder Solvate, und
- von 0,5 % bis 10 Gewichtsprozent DiC10C8 oder dessen Salze, Isomere oder Solvate,
wobei sich der Gewichtsprozentsatz auf das Gewicht des genannten Rhamnolipids, berechnet auf die Säureform (COOH), bezogen auf das Gesamtgewicht der Rhamnolipide in der Mischung, berechnet auf die Säureform (COOH), bezieht.

6. Die nichttherapeutische Verwendung nach einem beliebigen der Ansprüche 1 bis 5, wobei das mindestens eine Rhamnolipid in einem Bakterienextrakt enthalten ist.

7. Die nichttherapeutische Verwendung nach Anspruch 6, wobei das mindestens eine Rhamnolipid 40 Gew.-% bis 65 Gew.-% des Gesamttrockensubstanzgewichts des Bakterienextrakts ausmacht.

8. Die nichttherapeutische Verwendung nach Anspruch 6 oder 7, wobei der Bakterienextrakt aus einer Kultur von mindestens einem Stamm von *Pseudomonas aeruginosa* erhalten wird.

9. Die nichttherapeutische Verwendung nach einem beliebigen der Ansprüche 6 bis 8, wobei der Bakterienextrakt durch Fermentieren von mindestens einem Stamm von *Pseudomonas aeruginosa* in einem Kulturmedium, Abtrennen des Bakterienextrakts, der das mindestens eine Rhamnolipid umfasst, von den Bakterienzellen und gegebenenfalls zumindest teilweises Dehydratisieren, Sterilisieren, Reinigen, Mahlen und/oder Acylieren des Bakterienextrakts erhalten wird.

10. Die nichttherapeutische Verwendung nach einem beliebigen der Ansprüche 1 bis 9, wobei das mindestens eine Rhamnolipid in einer kosmetischen Zusammensetzung enthalten ist, die ferner einen kosmetisch akzeptablen Träger umfasst.

11. Die nichttherapeutische Verwendung nach Anspruch 10, wobei das mindestens eine Rhamnolipid in der kosmetischen Zusammensetzung in einer Konzentration von 0,0001 Gew.-% bis 30 Gew.-% bezogen auf das Gesamtgewicht der Zusammensetzung enthalten ist.

12. Die nichttherapeutische Verwendung nach einem beliebigen der Ansprüche 1 bis 11, wobei die genannte Verwendung topisch ist.

13. Zusammensetzung, umfassend eine Mischung von Rhamnolipiden,
wobei die Mischung Folgendes umfasst:
- von 31 Gew.-% bis 39 Gew.-% MonoC10C10 oder dessen Salze, Isomere oder Solvate,
- von 45 Gew.-% bis 55 Gew.-% DiC10C10 oder dessen Salze, Isomere oder Solvate,
- von 5 Gew.-% bis 15 Gew.-% DiC10C12 oder dessen Salze, Isomere oder Solvate, und
- von 0,5 Gew.-% bis 10 Gew.-% DiC10C8 oder dessen Salze, Isomere oder Solvate,
wobei sich der Gewichtsprozentsatz auf das Gewicht des genannten Rhamnolipids, berechnet auf die Säureform (COOH), bezogen auf das Gesamtgewicht der Rhamnolipide in der Mischung, berechnet auf die Säureform (COOH), bezieht.

## Revendications

1. Utilisation non thérapeutique d'au moins un rhamnolipide ou d'une composition cosmétique le comprenant, pour le traitement cosmétique des rougeurs cutanées induites par la consommation d'aliments ou de boissons chaudes ou alcoolisées, par une faible humidité relative, par l'exposition de la peau à des vents violents ou à des courants d'air, par une contrainte mécanique ou par l'application de tensioactifs.

2. L'utilisation non thérapeutique selon la revendication 1, dans lequel ledit au moins un rhamnolipide est au moins un rhamnolipide de formule (I) 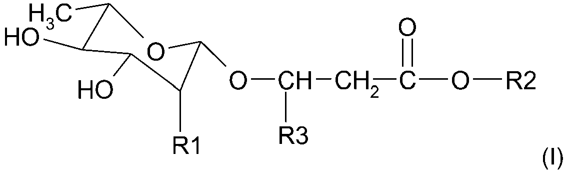 dans lequel
R₁ est choisi dans le groupe constitué de H et d'α-L-rhamnopyranosyle,
R₂ est choisi dans le groupe constitué de H, d'un alkyle linéaire ou ramifié en C₁-C₆, de -CH(R₄)-CH₂-COOH et de CH(R₄)-CH₂-COOR₅,
R₃ est -(CH₂)ₓCH₃, où x est un entier de 4 à 12, de préférence x est 6,
R₄ est -(CH₂)_{y}CH₃, où y est un entier de 1 à 10, de préférence y est 4, 6 ou 8, et
R₅ est un alkyle linéaire ou ramifié en C₁-C₆, de préférence -CH₃.

3. L'utilisation non thérapeutique selon la revendication 1 ou 2, dans lequel ledit au moins un rhamnolipide est un mélange de rhamnolipides, ledit mélange comprenant au moins un di-rhamnolipide de formule (II) 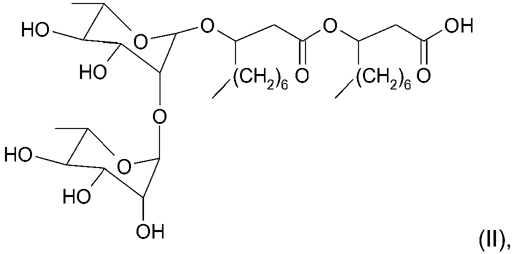 appelé DiC10C10,
ou ses sels, isomères ou solvates, et au moins un mono-rhamnolipide de formule (III) 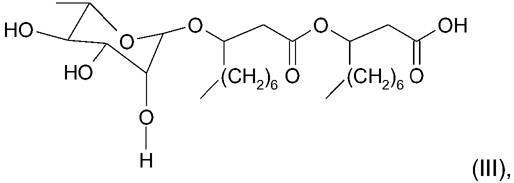 appelé MonoC10C10,
ou ses sels, isomères ou solvates.

4. L'utilisation non thérapeutique selon la revendication 3, dans lequel ledit au moins un rhamnolipide est un mélange comprenant MonoC10C10 ou ses sels, isomères ou solvates, DiC10C10 ou ses sels, isomères ou solvates, le di-rhamnolipide de formule (IV) 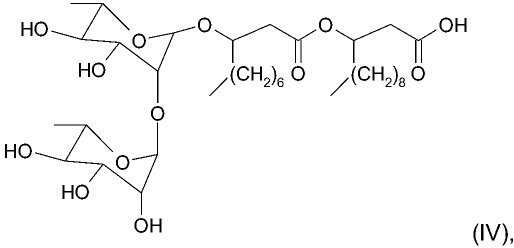 appelé DiC10C12,
ou ses sels, isomères ou solvates,
et le di-rhamnolipide de formule (V) 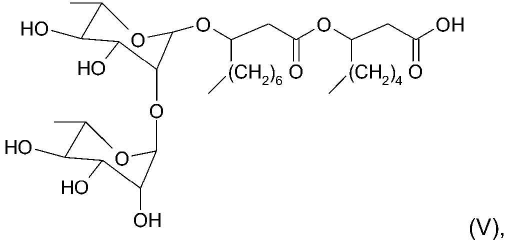 appelé DiC10C8, ou ses sels, isomères ou solvates.

5. L'utilisation non thérapeutique selon l'une quelconque des revendications 3 à 4, dans lequel le mélange comprend :
- de 30 % à 40 % en poids de MonoC10C10 ou de ses sels, isomères ou solvates,
- de 45 % à 55 % en poids de DiC10C10 ou de ses sels, isomères ou solvates,
- de 5 % à 15 % en poids de DiC10C12 ou de ses sels, isomères ou solvates, et
- de 0,5 % à 10 % en poids de DiC10C8 ou de ses sels, isomères ou solvates,
dans lequel le pourcentage en poids se rapporte au poids dudit rhamnolipide calculé sous la forme acide (COOH) par rapport au poids total des rhamnolipides dans le mélange calculé sous la forme acide (COOH).

6. L'utilisation non thérapeutique selon l'une quelconque des revendications 1 à 5, dans lequel l'au moins un rhamnolipide est inclus dans un extrait bactérien.

7. L'utilisation non thérapeutique selon la revendication 6, dans lequel l'au moins un rhamnolipide représente 40 % à 65 % en poids du poids total de matière sèche de l'extrait bactérien.

8. L'utilisation non thérapeutique selon la revendication 6 ou 7, dans lequel l'extrait bactérien est obtenu à partir d'une culture d'au moins une souche de *Pseudomonas aeruginosa.*

9. L'utilisation non thérapeutique selon l'une quelconque des revendications 6 à 8, dans lequel l'extrait bactérien est obtenu en faisant fermenter au moins une souche de *Pseudomonas aeruginosa* dans un milieu de culture, en séparant l'extrait bactérien comprenant l'au moins un rhamnolipide des cellules bactériennes, et facultativement en déshydratant au moins partiellement, en stérilisant, en purifiant, en broyant et/ou en acylant l'extrait bactérien.

10. L'utilisation non thérapeutique selon l'une quelconque des revendications 1 à 9, dans lequel l'au moins un rhamnolipide est contenu dans une composition cosmétique comprenant en outre un véhicule cosmétiquement acceptable.

11. L'utilisation non thérapeutique selon la revendication 10, dans lequel l'au moins un rhamnolipide est compris dans la composition cosmétique à une concentration de 0,0001 % à 30 % en poids par rapport au poids total de la composition.

12. L'utilisation non thérapeutique selon l'une quelconque des revendications 1 à 11, dans lequel ladite utilisation est topique.

13. Composition comprenant un mélange de rhamnolipides,
dans lequel le mélange comprend :
- de 31% à 39% en poids de MonoC10C10 ou de ses sels, isomères ou solvates,
- de 45% à 55% en poids de DiC10C10 ou de ses sels, isomères ou solvates,
- de 5% à 15% en poids de DiC10C12 ou de ses sels, isomères ou solvates, et
- de 0.5% à 10% en poids de DiC10C8 ou de ses sels, isomères ou solvates,
dans lequel le pourcentage en poids se rapporte au poids dudit rhamnolipide calculé sous la forme acide (COOH) par rapport au poids total des rhamnolipides dans le mélange calculé sous la forme acide (COOH).
